# EUROPEAN PATENT APPLICATION

(11) **EP 4 807 681 A2**
(43) Date of publication of application: **16.09.2026**
(21) Application number: 26163832.4
(22) Date of filing: 11.03.2026
(51) Int. Cl.: G06T 15/08, G06T 15/50, G16H 50/00

(54) **ENHANCING ISOSURFACES USING VIRTUAL LIGHT SOURCES IN MEDICAL IMAGES**

(30) Priority: 13.03.2025 US 202519078666
(71) Applicant: Siemens Medical Solutions USA, Inc., Malvern, PA 19355 (US)
(72) Inventor: VARCHOLA, Andrej, 040 11 Kosice (SK); SMITH-CASEM, Mervin Mencias, Issaquah, WA, 98029-7002 (US); KARIMOV, Alexey, 1210 Wien (AT)
(74) Representative: HKW Intellectual Property PartG mbB

(57) **Abstract**

A framework for enhancing isosurfaces using virtual light sources in medical images. The framework positions (204) one or more virtual light sources within the structure of interest in medical image data. Illumination effects (206) generated by the one or more virtual light sources are determined. Isosurface rendering of the medical image data using the illumination effects is then performed (208) to generate a rendered image for display.

## Description

### TECHNICAL FIELD

The present disclosure generally relates to medical imaging, and more particularly, to a framework for enhancing isosurfaces using virtual light sources in medical images.

### BACKGROUND

Diagnostic medical modalities, such as computed tomography (CT), magnetic resonance (MR) and ultrasound, may be used to acquire detailed volumetric medical images. The medical images depict three-dimensional (3D) anatomical structures, such as internal organs and tumors. Traditionally, cutting planes have been employed to navigate through volumetric data by "cutting" through the volume to reveal internal structures. This method allows for direct inspection of specific sections of the volume, but does not inherently improve the overall depth perception or differentiation of structures throughout the entire volume. It provides a more mechanical means of exploration rather than enhancing the visual representation for depth and boundary clarity.

In another traditional approach, the challenge of distinguishing between cavities and other structures within volume data is addressed by image segmentation methods. These methods involve processing the image to obtain segmentation masks, which differentiate various structures based on intensity, texture, or other criteria. This approach allows clinicians to identify and isolate specific areas of interest, facilitating the analysis of complex anatomical and pathological features. However, segmentation requires significant computational effort and expert intervention to accurately delineate boundaries, often struggling with overlapping structures or noisy data.

Utilizing gradients to encode depth information - either as a function of distance from the camera plane or from an external directional light source - has been a common technique to add a sense of depth to rendered images. While effective in providing a visual cue for depth, this approach can still struggle with complex internal structures, particularly in noisy or densely structured volumes. The depth cueing relies on visual gradients to infer depth, which may not be as effective in distinguishing closely packed or overlapping structures.

### SUMMARY

Described herein is a framework for enhancing isosurfaces using virtual light sources in medical images. The framework positions one or more virtual light sources within the structure of interest in medical image data. Illumination effects generated by the one or more virtual light sources are determined. Isosurface rendering of the medical image data is then performed using the illumination effects to generate a rendered image for display.

### BRIEF DESCRIPTION OF THE DRAWINGS

A more complete appreciation of the present disclosure and many of the attendant aspects thereof will be readily obtained as the same becomes better understood by reference to the following detailed description when considered in connection with the accompanying drawings.
FIG. 1 is a block diagram illustrating an exemplary imaging system;
FIG. 2 shows an exemplary method of medical image visualization;
FIG. 3 shows an exemplary image of the heart;
FIG. 4a show an exemplary slice of a light volume;
FIG. 4b shows another exemplary slice of a light volume;
FIG. 5 shows an exemplary rendered image of a heart; and
FIG. 6 shows another exemplary rendered image of a heart.

### DETAILED DESCRIPTION

In the following description, numerous specific details are set forth such as examples of specific components, devices, methods, etc., in order to provide a thorough understanding of implementations of the present framework. It will be apparent, however, to one skilled in the art that these specific details need not be employed to practice implementations of the present framework. In other instances, well-known materials or methods have not been described in detail in order to avoid unnecessarily obscuring implementations of the present framework. While the present framework is susceptible to various modifications and alternative forms, specific embodiments thereof are shown by way of example in the drawings and will herein be described in detail. It should be understood, however, that there is no intent to limit the invention to the particular forms disclosed, but on the contrary, the intention is to cover all modifications, equivalents, and alternatives falling within the spirit and scope of the invention. Furthermore, for ease of understanding, certain method steps are delineated as separate steps; however, these separately delineated steps should not be construed as necessarily order-dependent in their performance.

Unless stated otherwise as apparent from the following discussion, it will be appreciated that terms such as "segmenting," "generating," "registering," "determining," "aligning," "positioning," "processing," "computing," "selecting," "estimating," "detecting," "tracking" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, implementations of the present framework are not described with reference to any particular programming language. It will be appreciated that a variety of programming languages may be used.

Isosurface rendering is a technique for visualizing three-dimensional (3D) structures within volume data by implicitly extracting an isosurface, which is a surface that passes through all data points that have the same scalar value. While effective in certain scenarios, this method encounters difficulties when applied to noisy volume data or structures with complex internal features such as cavities, vessel walls, or fluid-filled organs. The primary challenge lies in the inability to clearly differentiate between overlapping structures and noise artifacts, leading to a rendered image where the boundaries of 3D shapes are not distinct.

One aspect of the present framework enhances visualization of medical images by visual segmentation of internal structures using one or multiple internal virtual light sources combined with isosurface rendering for improved depth perception and boundary distinction. The present framework enables positioning of one or multiple internal virtual light sources for illumination within the anatomical structure(s) of interest. A light volume or cache may be used for storing illumination effects. Alternatively, the illumination effects may be computed directly from each virtual light source.

The present framework applies virtual light sourcing techniques to the challenge of isosurface volume rendering in medical imaging. The virtual light sources may be combined with isosurface rendering for enhanced boundary distinction of internal cavities. Isosurface or silhouette rendering modes are used in a variety of medical imaging products, including ultrasound scanners. However, traditional methods encounter difficulties when applied to noisy volume data or structures with complex internal features, such as cavities, vessel walls, or fluid-filled organs. The present framework combines virtual light sources that are strategically positioned inside of cavities to enhance only internal structures of interest that will otherwise be hardly differentiable because of overlapping structures and noise artifacts. The present framework may be applied to a wide range of applications, including, but not limited to, cardiac imaging, obstetric imaging, gynecology, urology, and abdominal imaging. These and other exemplary advantages and features will be described in more details in the following description.

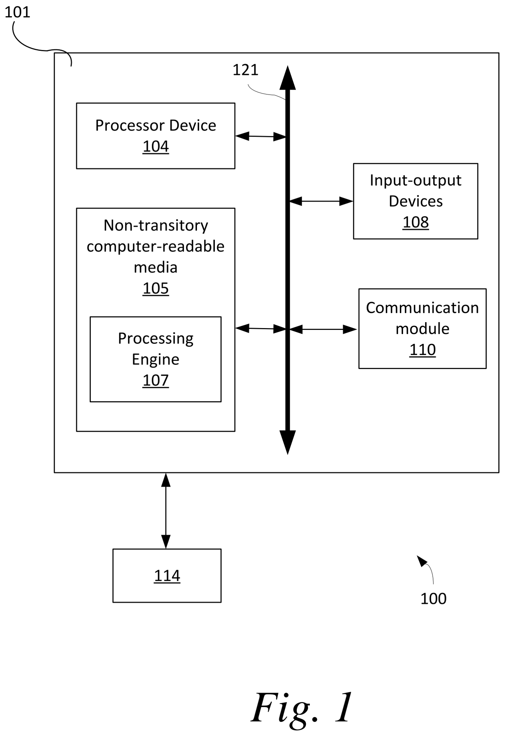

FIG. 1 is a block diagram illustrating an exemplary medical imaging system 100. In some implementations, system 100 includes a computer system 101 coupled to a medical imaging device 114. Computer system 101 includes a processor device 104 coupled to one or more non-transitory computer-readable media 105 (e.g., computer storage or memory device), input-output devices 108 (e.g., monitor, mouse, touchpad or keyboard) and communication module 110. Processor device 104 may include, for example, a central processing unit (CPU), a graphics processing unit (GPU), field-programmable gate array (FPGA), or a combination thereof. Computer system 101 may further include support circuits such as a cache, a power supply or battery, clock circuits, and a communications bus (not shown). Various other peripheral devices, such as client devices (e.g., workstations) and additional data storage devices and printing devices, may also be connected to the computer system 101.

The present technology may be implemented in various forms of hardware, software, firmware, special purpose processors, or a combination thereof, either as part of the microinstruction code or as part of an application program or software product, or a combination thereof, which is executed via the operating system. In some implementations, the techniques described herein are implemented as computer-readable program code tangibly embodied in one or more non-transitory computer-readable media 105. In particular, the present techniques may be implemented by a processing engine 107. Non-transitory computer-readable media 105 may include random access memory (RAM), read-only memory (ROM), magnetic floppy disk, flash memory, and other types of memories, or a combination thereof. The computer-readable program code is executed by processor device 104 to process data acquired by, for example, medical imaging device 114. The computer-readable program code is not intended to be limited to any particular programming language and implementation thereof. It will be appreciated that a variety of programming languages and coding thereof may be used to implement the teachings of the disclosure contained herein. The same or different computer-readable media 105 may be used for storing a database, including, but not limited to, image datasets, a knowledge base, individual subject data, medical records, diagnostic reports (or documents) for subjects, or a combination thereof.

Communication module 110 enables the computer system 101 to communicate with external systems and/or networks. In some implementations, communication module 110 includes a high-speed digital interface, such as Thunderbolt^{™}, universal serial bus (USB), multi-Gigabit Ethernet, optical fiber, waveguide technology, or a wireless interface. Other types of interfaces are also useful. In some implementations, communication module 110 includes wireless signal transceiver that communicates signals using a common communication protocol, such as Global System for Mobile Communications (GSM), WIFI, Bluetooth, Zigbee, LoRa, and TCP/IP.

Medical imaging device 114 is a radiological imaging device that acquires medical image data that reveals internal structures hidden by skin and bones of the subject. Such medical imaging device 114 may use technologies of X-ray radiography, computed tomography (CT), magnetic resonance (MR) imaging, ultrasound, endoscopy, elastography, tactile imaging, thermography, medical photography, nuclear medicine functional imaging techniques (e.g., positron emission tomography (PET), single-photon emission computed tomography (SPECT)), or a combination thereof.

It is to be further understood that, because some of the constituent system components and method steps depicted in the accompanying figures can be implemented in software, the actual connections between the systems components (or the process steps) may differ depending upon the manner in which the present framework is programmed. Given the teachings provided herein, one of ordinary skill in the related art will be able to contemplate these and similar implementations or configurations of the present framework.

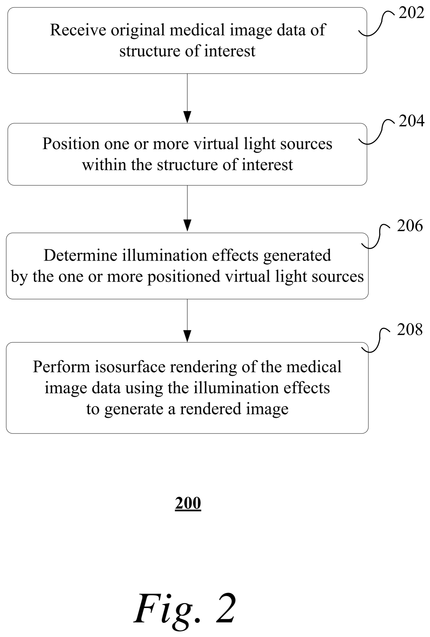

FIG. 2 shows an exemplary method 200 of medical image visualization. It should be understood that the steps of the method 200 may be performed in the order shown or a different order. Additional, different, or fewer steps may also be provided. Further, the method 200 may be implemented with system 100 of FIG. 1, a different system, or a combination thereof.

At 202, processing engine 107 receives original medical image data of at least one structure of interest. The original medical image data may be provided from a memory, a medical scanner 114, sensors, and/or other source. The original medical image data may be formatted as voxels. Each voxel may be represented by 3D location (e.g., x, y, z) and an intensity, scalar, or other information. A medical scanner 114 may provide the original medical image data, such as a medical dataset representing a 3D region of the structure of interest in a patient. Other type of medical image data may also be obtained, such as computed tomography, magnetic resonance, positron emission tomography, single photon emission computed tomography, or another scan modality. Medical image data representing a 3D volume may be loaded as a medical dataset. The structure of interest may be any anatomical structure (e.g., cardiac structure such as mitral valve, aortic valve, atria, ventricles) that is identified for further study.

A user interface may be provided to enable a user to select one or more structures of interest in the medical image data. In some implementations, an artificial intelligence (AI)-based analytical algorithm is independently applied to automatically detect and segment structures of interest. In other implementations, particularly in some scanning scenarios, such as 3D transesophageal echocardiography (TEE), the scanning protocols provide fairly consistent positions of structures of interest within the medical data.

Returning to FIG. 2, at 204, processing engine 107 positions one or more virtual light sources within the structure of interest. Virtual light sources simulate the effect of light rays emitted by light sources to enhance depth perception in volume-rendered images for visual segmentation. Virtual light sources may be, for example, a point light source, a spot light source, a directional light source, an area light source, an ambient light source, or a combination thereof. A point light source emits light from a single point in all directions, with the intensity of the light decreasing with distance, while a spot light source produces a directed cone of light. Other types of virtual light sources are also useful. The shadowing effects introduced by virtual light sources can significantly enhance the viewer's ability to perceive depth in the rendered image, making it easier to understand the spatial relationships between different structures.

In some implementations, processing engine 107 automatically determines the positions and/or assigns colors to the virtual light sources based on specific characteristics of the medical image data. To determine the positions, processing engine 107 may analyze the structure of interest in the medical image data to identify one or more features for highlighting and automatically place lights within these features. A feature may be a volume that is enclosed or surrounded by an inner wall. The feature may be, for example, a cavity, a lumen, a tubular structure, or a combination thereof. For instance, the virtual light source may be positioned at or near the centroid of a cavity (e.g., heart chamber), or along a centerline of a tubular structure (e.g., artery). Multiple virtual light sources may be positioned along a centerline of the significant feature. To determine the centerline, skeletonization techniques, such as thinning algorithms, may be used. Additionally, multiple virtual light sources may be positioned in different elements of the structure of interest. These multiple virtual light sources may be assigned different colors to enable unique illumination of each feature.

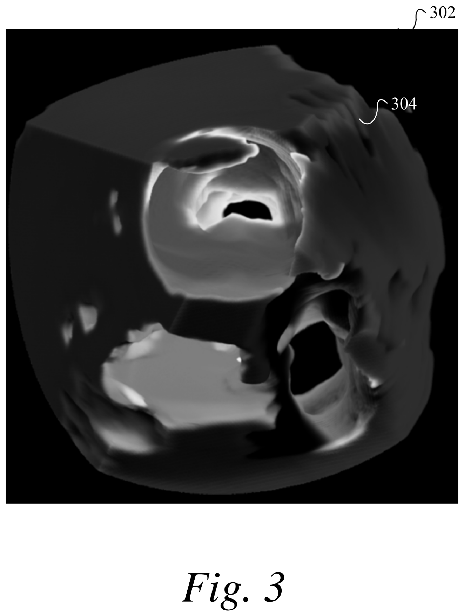

FIG. 3 shows an exemplary image 302 of the heart. Five virtual point lights have been automatically positioned in chambers and arteries of the heart 304. Each virtual light source has been assigned a different color. This differentiation facilitates distinction between boundaries of various elements within the heart, thereby addressing the primary challenge identified with isosurface rendering, which is the inability to clearly differentiate between overlapping structures and noise artifacts. This method may significantly reduce the time and expertise required to generate the visualization, thereby making advanced imaging techniques more accessible to a broader range of users.

Additionally, or in combination thereof, processing engine 107 generates a user interface that enables a user (e.g., clinical expert) to interactively provide user input to position or adjust the automatically determined positions of the virtual light sources in real-time. The user interface may also enable the user to assign one or more colors to the one or more virtual light sources. The user input may be automatically optimized. For example, the user-provided position may be automatically centered at a centroid within a cavity. This method provides a high degree of control over the visualization, enabling experts to highlight specific areas based on their knowledge and needs. By placing virtual light sources on the MultiPlanar Reconstruction (MPR) image or directly within the 3D scene, users can tailor the lighting to accentuate certain cavities or structures, enhancing the interpretability of complex data and effectiveness of visualization.

In some implementations, ray profiling (or ray profile analysis) is performed to aid the placement of virtual light sources in, for example, click-on 3D views. Ray profiling is a technique that analyzes data values from the original medical image data (e.g., ultrasound data) encountered during ray-tracing. Such technique may also collect information on accumulated color and/or occlusion along the ray. The ray profile may be analyzed to identify peaks or valleys in the data values. For example, valleys in the ray profile may correspond to cavities or empty space in the volume, while peaks in the ray profile may correspond to the interfaces between different tissues. Virtual light sources may be positioned in features (e.g., cavities) identified from the ray profile. The start of the ray for the ray profile analysis may be defined by the user selecting (e.g., clicking on) a location in, for example, the MPR view or 3D view. 3D view ray profile analysis may be performed from several selected points and viewing angles to confirm cavities.

Returning to FIG. 2, at 206, processing engine 107 determines illumination effects generated by the one or more positioned virtual light sources. Illumination effects enable the effect of enhanced inner isosurface to be achieved. Illumination effects may be determined by simulating the effect of the one or more virtual light sources by adding shadows, highlights and/or colors to provide illumination from the virtual light source within the structure of interest.

In some implementations, the illumination effects by each virtual light source are determined and stored in a light volume (or light cache) to speed up rendering. A light volume may be a type of texture that is overlaid on the structure of interest to provide light illumination information for rendering an image. The light illumination effects may include, for example, light intensity per color channel, light direction, occlusion, or a combination thereof. The light intensity may be subject to absorption by voxels of the original medical image.

This light volume functions as a map or cache, recording how each virtual light source impacts the volumetric medical image data, thereby facilitating real-time adjustments and rendering. The selective enhancement of illuminated elements in the structure of interest ensures that attention is drawn to areas of interest without overwhelming the viewer with unnecessary detail. The light volume may be pre-computed or generated in real-time for each frame of the medical image volume. Additionally, the light volume may be re-computed in response to a change in one or more parameters. Such parameters may include, but are not limited to, lighting setup parameters, transfer functions, isosurface parameters, medical image volume, or a combination thereof.

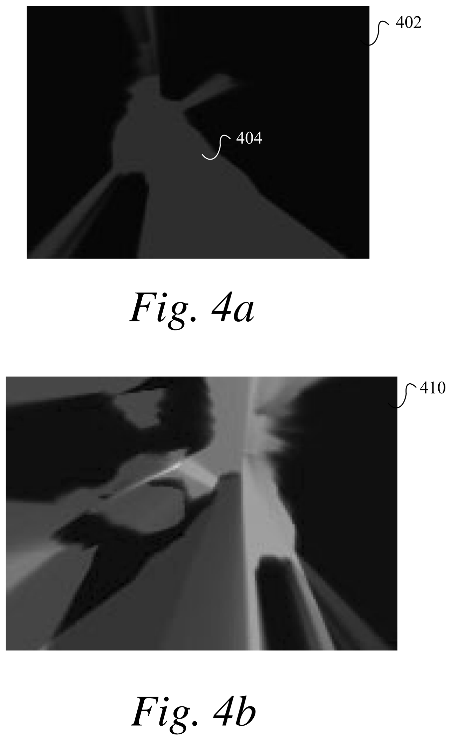

FIG. 4a show an exemplary slice 402 of a light volume. A blue virtual light source is positioned in the heart chamber 404 of cardiac ultrasound image data. FIG. 4b shows another exemplary slice 410 of a light volume. Five virtual point lights with different colors (pink, red, green, blue, yellow) are positioned in different chambers and arteries of the heart in the ultrasound image data.

Returning to FIG. 2, at 208, processing engine 107 performs isosurface rendering of the medical image data using the illumination effects to generate a rendered image for display. Isosurface rendering is a volume rendering technique used to visualize surfaces within volume data that meet a specific threshold value, effectively creating a 3D representation of the area of interest. See, for example, M. Levoy, "Display of surfaces from volume data," in IEEE Computer Graphics and Applications, vol. 8, no. 3, pp. 29-37, May 1988, which is herein incorporated by reference. Isosurface rendering utilizes gradient-based surface shading to calculate opacity levels for every sample or voxel.

In some implementations, the light volume is used during the isosurface rendering to render the images for display. Alternatively, the illumination effects are directly computed. Emission-absorption light transport equations may be used to model the illumination effects. In some implementations, the light volume is computed from the original volume data and used at the isosurface rendering to highlight differentiating isosurfaces that enclose different light sources. Combined with placement of light sources inside cavities surrounded by isosurfaces, some implementations arrive at highlighted isosurfaces differentiated by connectivity. In other implementations, the light volume is computed from and used at the isosurfaces. By integrating virtual light sources into this process, the present framework leverages the strength of isosurface rendering to depict boundaries and surfaces while overcoming its limitations in depth perception and differentiation of internal structures.

In some implementations, composition of illuminated volume samples is done using, for example, alpha blending. Alpha blending is the process of combining one image with a background to create the appearance of partial or full transparency. Each voxel may have an additional numeric value stored in its alpha channel, with an alpha value ranging from 0 to 1. An alpha value of 0 means that the voxel is fully transparent and the color in the voxel beneath will show through. An alpha value of 1 means that the voxel is fully opaque. Alpha channel values may be derived from the original medical image data utilizing transfer functions and/or segmentation masks. The composition of illuminated volume samples produces one volume image and has to be remade each time the transfer functions and/or segmentation masks change.

The isosurface rendering may be performed using an emission-absorption light transport equation that involves absorption coefficients of data. An absorption coefficient specifies how much light is absorbed per unit of volume. To derive absorption coefficients, a transport color transfer function may be used to map an alpha channel to a color. The composition of several light sources into one light volume may be performed by addition and/or subtraction of computed absorption coefficient values. Absorption by characteristics derived from the medical image data may be modeled as follows: $C 1 = \left(1-A0*IA\right) * C 0$ wherein C0 represents the incident light, C1 represents the exitant light, IA represents absorption coefficients per red/green/blue (RGB) color channel, and A0 is the alpha value of the tissue sample. The mapping of the color C0 and A0 may be assigned using a ramp transfer function defined by center and width, wherein the center corresponds to the iso-surface value.

For the purpose of optimizing performance, the light volume computation may be performed on a scaled-down (or downsampled) representation of the original medical image data. The scaled-down representation provides smoother transitions between different features of the volume at the cost of reduced details. Since illumination is diffuse in nature, no noticeable image degradation occurs with the scaled-down representation. It should be noted that composition of illuminated volume samples and computation of light cache may be performed on different medical data sets without introducing image artifacts. For example, composition of illuminated volume samples may be performed on original medical image data, while computation of the light cache may be performed on scaled-down representation of the original medical image data.

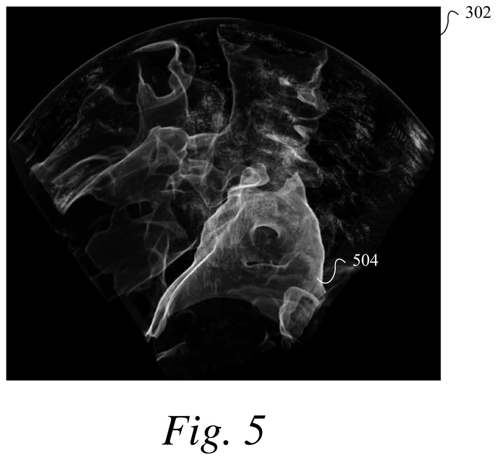

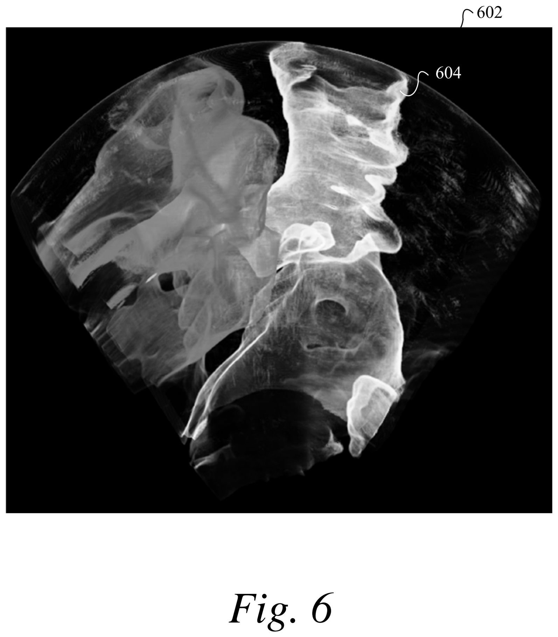

FIG. 5 shows an exemplary rendered image 502 of a heart. Isosurface 504 is enhanced with a virtual point light source positioned inside a cardiac chamber in ultrasound volume data. FIG. 6 shows another exemplary rendered image 602 of a heart. Isosurface rendering is enhanced with five virtual point lights positioned in chambers and arteries of the heart 604. Each virtual point light is assigned a different color. By positioning lights within the image volume and using a light illumination effect, it allows for a dynamic and spatially aware method to enhance visual cues for depth and structure differentiation. The framework goes beyond simple visual gradients or sectional views, offering a way to illuminate and shade internal structures in a manner that can highlight boundaries and depth relationships more clearly. The assignment of different colors to virtual light sources positioned within different cavities or structures adds an additional layer of differentiation, potentially making it easier to distinguish between various elements within the volume.

The framework's ability to enhance visualization of internal structures using virtual light sources and iso-surface rendering can improve diagnostic imaging in several key areas. For example, for cardiac applications, especially four-dimensional (4D) Intracardiac Echocardiography (ICE) and procedures involving structural hearts and ablations, precise visualization of the heart's internal structures is crucial. This framework can allow for better delineation of cardiac chambers, valves, and abnormal pathways during interventional procedures, thereby improving safety and outcomes. Useful information for structural and diagnostic cardiac imaging may also be provided. The visualization of different chambers and anatomical structures is enhanced for ablations, valves, and Left Atrial Appendage devices, with the virtual light sources offering greater differentiation of various tissue details. Furthermore, chamber, valve and vessel modeling enable diagnostic evaluations of a wide range of pathologies, including investigations of dilatation, diverticula, aneurysms, shunts, single ventricle, Ebstein's anomaly, Tetralogy of Fallot, perforations, perivalvular leaks, and/or transposition of great vessels. This can aid in the guidance for interventional procedures and allows for a better comparison for multimodality imaging techniques, such as CT and MR, thereby enhancing the diagnostic process.

In obstetric imaging, the present framework provides the ability to clearly visualize fetal cardiac structures, including ventricles and atria, and identify any associated structural abnormalities. This framework can significantly enhance early diagnosis and monitoring of fetal health, especially in complex scenarios like twin pregnancies or placental anomalies. Techniques such as color Doppler imaging for assessing conditions like placenta accreta or percreta, which require detailed visualization of blood flow and tissue invasion, will benefit greatly from enhanced depth and boundary distinction.

In gynaecologic imaging, saline sonohysterography relies on clear imaging of the uterine cavity to diagnose anomalies and pathologies. The improved visualization offered by this framework can lead to better identification and characterization of polyps, fibroids, and other uterine conditions without interference from overlying structures. Additionally, it can provide clearer images in cases of pelvic inflammatory disease, differentiating between conditions like hydrosalpinx or pyosalpinx more effectively.

For urological applications, the present framework is particularly useful in highlighting bladder wall pathologies, such as transitional cell carcinoma (TCC), which often occurs at the bladder base. The precise visualization of the extent of diseases like endometriosis, when it invades the bladder wall, or the detailed structure of the bladder wall in cases of placenta percreta, can improve both diagnostic accuracy and the planning of surgical interventions.

Abdominal imaging, particularly for gallbladder pathologies including choledochal cysts, can benefit from enhanced visualization enabled by the present framework. Accurate delineation of cystic structures and their relationships with surrounding tissues can aid in diagnosis, surgical planning and patient management.

The following is a list of non-limiting illustrative embodiments disclosed herein:

Illustrative embodiment 1. A method of medical image visualization, comprising: receiving medical image data of at least one structure of interest; positioning one or more virtual light sources within the structure of interest; determining illumination effects generated by the one or more virtual light sources; and performing isosurface rendering of the medical image data using the illumination effects to generate a rendered image for display.

Illustrative embodiment 2. The method of illustrative embodiment 1 wherein the one or more virtual light sources comprise a point light source, a spot light source, a directional light source, an area light source, an ambient light source, or a combination thereof.

Illustrative embodiment 3. The method of any one of illustrative embodiments 1-2 wherein positioning the one or more virtual light sources within the structure of interest comprises: identifying one or more features in the at least one structure of interest; and positioning the one or more virtual light sources within the one or more features.

Illustrative embodiment 4. The method of illustrative embodiment 3 wherein the one or more features comprise a cavity, a lumen, a tubular structure or a combination thereof.

Illustrative embodiment 5. The method of illustrative embodiment 3 wherein positioning the one or more virtual light sources within the one or more features comprises positioning the one or more virtual light sources at or near a centroid of the one or more features.

Illustrative embodiment 6. The method of illustrative embodiment 3 wherein positioning the one or more virtual light sources within the one or more features comprises positioning multiple virtual light sources along a centerline of the one or more features.

Illustrative embodiment 7. The method of illustrative embodiment 6 further comprises assigning different colors to the multiple virtual light sources.

Illustrative embodiment 8. The method of any one of illustrative embodiments 1-7 wherein positioning the one or more virtual light sources within the structure of interest comprises generating a user interface to receive user input of one or more positions of the one or more virtual light sources.

Illustrative embodiment 9. The method of illustrative embodiment 8 further comprising optimizing the user input of the one or more positions.

Illustrative embodiment 10. The method of any one of illustrative embodiments 1-9 wherein positioning the one or more virtual light sources within the structure of interest comprises generating a user interface to receive user input of one or more colors of the one or more virtual light sources.

Illustrative embodiment 11. The method of any one of illustrative embodiments 1-10 wherein positioning the one or more virtual light sources within the structure of interest comprises performing ray profiling to identify one or more features to place the one or more virtual light sources in.

Illustrative embodiment 12. The method of any one of illustrative embodiments 1-11 wherein determining the illumination effects generated by the one or more virtual light sources storing the illumination effects in a light volume.

Illustrative embodiment 13. The method of illustrative embodiment 12 further comprising re-computing the light volume in response to a change in one or more parameters.

Illustrative embodiment 14. The method of any one of illustrative embodiments 1-13 wherein the illumination effects comprise light intensity per color channel, light direction, occlusion, or a combination thereof.

Illustrative embodiment 15. The method of any one of illustrative embodiments 1-14 wherein performing the isosurface rendering of the medical image data comprises performing a light compositing step to add light color to the rendered image.

Illustrative embodiment 16. A system, comprising: a non-transitory memory device for storing computer readable program code; and a processor device in communication with the non-transitory memory device, the processor device being operative with the computer readable program code to perform steps including receiving medical image data of at least one structure of interest, positioning one or more virtual light sources within the structure of interest, determining illumination effects generated by the one or more virtual light sources, and performing isosurface rendering of the medical image data using the illumination effects to generate a rendered image for display.

Illustrative embodiment 17. The system of illustrative embodiment 16 wherein the processor device is operative with the computer readable program code to position the one or more virtual light sources within the structure of interest by identifying one or more features in the at least one structure of interest, and positioning the one or more virtual light sources within the one or more features.

Illustrative embodiment 18. The system of illustrative embodiment 17 wherein the one or more features comprise a cavity, a lumen, a tubular structure or a combination thereof.

Illustrative embodiment 19. The system of illustrative embodiment 17 wherein the processor device is operative with the computer readable program code to position the one or more virtual light sources within the one or more features by positioning the one or more virtual light sources at or near a centroid of the one or more features.

Illustrative embodiment 20. One or more non-transitory computer-readable media comprising computer-readable instructions, that when executed by a processor device, cause the processor device to perform steps comprising: receiving medical image data of at least one structure of interest; positioning one or more virtual light sources within the structure of interest; determining illumination effects generated by the one or more virtual light sources; and performing isosurface rendering of the medical image data using the illumination effects to generate a rendered image for display.

While the present framework has been described in detail with reference to exemplary embodiments, those skilled in the art will appreciate that various modifications and substitutions can be made thereto without departing from the spirit and scope of the invention as set forth in the appended claims. For example, elements and/or features of different exemplary embodiments may be combined with each other and/or substituted for each other within the scope of this disclosure and appended claims.

## Claims

1. A method (200) of medical image visualization, comprising:
receiving (202) medical image data of at least one structure of interest;
positioning (204) one or more virtual light sources within the structure of interest;
determining (206) illumination effects generated by the one or more virtual light sources; and
performing isosurface rendering (208) of the medical image data using the illumination effects to generate a rendered image for display.

2. The method (200) of claim 1 wherein the one or more virtual light sources comprise a point light source, a spot light source, a directional light source, an area light source, an ambient light source, or a combination thereof.

3. The method (200) of claim 1 or 2 wherein positioning (204) the one or more virtual light sources within the structure of interest comprises:
identifying one or more features in the at least one structure of interest; and
positioning the one or more virtual light sources within the one or more features.

4. The method (200) of claim 3 wherein the one or more features comprise a cavity, a lumen, a tubular structure or a combination thereof.

5. The method (200) of claim 3 or 4 wherein positioning (204) the one or more virtual light sources within the one or more features comprises positioning the one or more virtual light sources at or near a centroid of the one or more features.

6. The method (200) of one of claims 3 to 5 wherein positioning (204) the one or more virtual light sources within the one or more features comprises positioning multiple virtual light sources along a centerline of the one or more features.

7. The method (200) of claim 6 further comprises assigning different colors to the multiple virtual light sources.

8. The method (200) of one of claims 1 to 7 wherein positioning (204) the one or more virtual light sources within the structure of interest comprises generating a user interface to receive user input of one or more positions of the one or more virtual light sources.

9. The method (200) of claim 8 further comprising optimizing the user input of the one or more positions.

10. The method (200) of one of claims 1 to 9 wherein positioning (204) the one or more virtual light sources within the structure of interest comprises generating a user interface to receive user input of one or more colors of the one or more virtual light sources.

11. The method (200) of one of claims 1 to 10 wherein determining (206) the illumination effects generated by the one or more virtual light sources comprises storing the illumination effects in a light volume.

12. The method (200) of claim 11 further comprising re-computing the light volume in response to a change in one or more parameters.

13. The method (200) of one of claims 1 to 12 wherein the illumination effects comprise light intensity per color channel, light direction, occlusion, or a combination thereof.

14. The method (200) of one of claims 1 to 13 wherein performing the isosurface rendering (208) of the medical image data comprises performing a light compositing step to add light color to the rendered image.

15. A system (101), comprising:
a non-transitory memory device (105) for storing computer readable program code; and
a processor device (104) in communication with the non-transitory memory device (105), the processor device (104) being operative with the computer readable program code to perform steps including
receiving (202) medical image data of at least one structure of interest,
positioning (204) one or more virtual light sources within the structure of interest,
determining (206) illumination effects generated by the one or more virtual light sources, and
performing isosurface rendering (208) of the medical image data using the illumination effects to generate a rendered image for display.
